# EUROPEAN PATENT SPECIFICATION

(11) **EP 1 210 603 B1**
(45) Date of publication and mention of the grant of the patent: **06.10.2010**
(21) Application number: 00955686.1
(22) Date of filing: 17.08.2000
(51) Int. Cl.: G01N 33/574

(54) **ANTI-EPHA2 ANTIBODIES AS A CANCER DIAGNOSTIC**
ANTI-EPHA2 ANTIKÖRPER ALS KREBSDIAGNOSTIKUM
ANTICORPS ANTI-EPHA2 UTILISES DANS LE DIAGNOSTIC DU CANCER

(30) Priority: 17.08.1999 US 149259 P
(43) Date of publication of application: 05.06.2002
(73) Proprietor: Purdue Research Foundation, West Lafayette, IN 47906 (US); GLAXO GROUP LIMITED, Greenford, Middlesex UB6 0NN (GB); THE UNIVERSITY OF NORTH CAROLINA AT CHAPEL HILL, Chapel Hill, NC 27599-4105 (US)
(72) Inventor: KINCH, Michael, Scott, Lafayette, IN 47905 (US); ZANTEK, Nicole, Dodge, Silver Spring, MD 20904 (US); KILPATRICK, Katherine, E, Chapel Hill, NC 27516 (US)
(74) Representative: Vossius & Partner
(86) International application number: PCT/US2000/022669
(87) International publication number: WO 2001/012840

(56) References cited:
- US-A- 5 457 048
- WALKER-DANIELS J ET AL: "Overexpression of the EphA2 tyrosine kinase in prostate cancer." PROSTATE, vol. 41, no. 4, 1 December 1999 (1999-12-01), pages 275-280, XP000978523 ISSN: 0270-4137
- ZANTEK NICOLE DODGE ET AL: "Identification of an adhesion-associated tyrosine kinase that is tightly regulated in breast cancer." MOLECULAR BIOLOGY OF THE CELL, vol. 8, no. SUPPL., November 1997 (1997-11), page 134A XP000979706 37th Annual Meeting of the American Society for Cell Biology;Washington, D.C., USA; December 13-17, 1997 ISSN: 1059-1524
- ZANTEK NICOLE DODGE ET AL: "Epithelial cell kinase (ECK/EPHA2) regulation in breast cancer." MOLECULAR BIOLOGY OF THE CELL, vol. 9, no. SUPPL., November 1998 (1998-11), page 134A XP000980380 38th Annual Meeting of the American Society for Cell Biology;San Francisco, California, USA; December 12-16, 1998 ISSN: 1059-1524
- ZANTEK N D ET AL: "Regulation of the EphA2 receptor tyrosine kinase by estrogen and myc." PROCEEDINGS OF THE AMERICAN ASSOCIATION FOR CANCER RESEARCH ANNUAL, vol. 40, March 1999 (1999-03), page 687 XP000978765 90th Annual Meeting of the American Association for Cancer Research;Philadelphia, Pennsylvania, USA; April 10-14, 1999, March, 1999 ISSN: 0197-016X
- KINCH MICHAEL S ET AL: "Identification of tyrosine phosphorylated adhesion proteins in human cancer cells." HYBRIDOMA, vol. 17, no. 3, June 1998 (1998-06), pages 227-235, XP000980398 ISSN: 0272-457X

## Description

### Field of The Invention

The present invention relates to diagnosis of metastatic disease. More particularly, this invention relates to reagents that can detect a specific epithelial cell tyrosine kinase that is overexpressed in metastatic tumor cells. Most particularly, this invention relates to reagents which bind to the epithelial cell tyrosine kinase, and the use of these reagents for cancer diagnosis.

### Background And Summary of The Invention

Cancer cell metastasis requires cellular capacity to 1) detach from a primary tumor, 2) migrate and invade through local tissues, 3) translocate to distant sites in the body (via lymph or blood), 4) colonize a foreign site, and 5) grow and survive in this foreign environment. All of these behaviors are linked to cell adhesions. Cell adhesions control the physical interactions of cells with their microenvironment. Cell adhesions also initiate signals that dictate tumor cell growth, death, and differentiation.

Various cancer cells, including breast cancer cells, are known to exhibit altered cell adhesion. As compared to normal breast epithelia, transformed human breast epithelial cells have decreased cell-cell contacts and increased interactions with the surrounding extracellular matrix. These changes facilitate increased detachment and migration of cancer cells away from cell colonies and are directly linked with alteration in tyrosine phosphorylation of cell membrane proteins. Tyrosine phosphorylation is a potent form of cell signal transduction, and alternation in levels of tyrosine phosphorylation is believed to be important for tumor cell invasiveness. Thus, regulation of tyrosine phosphorylation represents a promising target for therapeutic intervention against metastatic cancer. Tyrosine phosphorylation is controlled by cell membrane tyrosine kinases, and increased expression of tyrosine kinases is known to occur in metastatic cancer cells.

Identification of increased expression of cell membrane tyrosine kinases would aid in the diagnosis and treatment of metastatic diseases. One such tyrosine kinase is EphA2. A member of the Eph family of tyrosine kinases known as Ephrins, EphA2 is a transmembrane receptor tyrosine kinase with a cell-bound ligand. Although cloned a decade ago, see Lindberg, RA. and Hunter, T., "cDNA Cloning and Characterization of Eck, an Epithelial Cell Receptor Protein-tyrosine Kinase in the Eph/elk Family of Protein Kinases," Mol. Cell. Biol. 10 (12), 6316-6324 (1990), rather little is known about EphA2 function, largely because EphA2-specific antibodies previously have been difficult to generate.

Zantek et al. (November 1998), Mol. Biol. Cell Vol.9 (suppl.), page 134A discloses that, in breast epithelia, EphA2 is expressed, phosphorylated and enriched in cell-cell contacts in normal cells; not expressed in poorly invasive cancer cells; expressed and not phosphorylated at membrane ruffles in metastatic cells

To facilitate research on EphA2, an improved method for generating a panel of monoclonal antibodies specific for tyrosine phosphorylated proteins has been developed. Using this method, a multiplicity of EphA2 recognizing monoclonal antibodies has been generated. These antibodies have been used to show that EphA2 is overexpressed in metastatic breast, lung, colon, and prostate cells. Because EphA2 is expressed differently in normal and metastatic cells, EphA2-specific antibodies are useful in the diagnosis of metastatic disease. Antibodies produced by one particular hybridoma recognize an intracellular epitope of EphA2 and have been shown to be highly specific in binding to EphA2.

Thus, one aspect of this invention is a method according to claims 1 and 34. Also disclosed is a method for generating antibodies which recognize EphA2 intracellular epitopes. Another aspect of this invention is the use of EphA2-specific antibodies in the diagnosis of metastatic disease. Also disclosed is a diagnostic reagent specific for detecting EphA2, any fragment thereof, or DNA or RNA coding for the EphA2 protein. A further aspect of this invention is a kit comprising an antibody capable of specific binding to an epitope of EphA2 and means for detecting antibody-EphA2 binding.

Additional features of the present invention will become apparent to those skilled in the art upon consideration of the following detailed description of preferred embodiments exemplifying the best mode of carrying out the invention as presently perceive. .

### Brief Description of The Drawings

Fig. 1A-C show a series of western blots showing EphA2 expression in cell lines derived from human prostate cells;
Fig. 1A is a western blot showing EphA2 expression in various human prostate cancer cell lines;
Fig. 1B is a western blot showing EphA2 expression in human prostatic epithelial cell line MLC and expression in that cell line after transformation by oncogenic K-Ras or X-irradiation;
Fig. 1C is similar to Fig. 1B, except showing expression in human prostatic epithelial cell line 267B1 and expression in that cell line after transformation by oncogenic K-Ras or X-irradiation;
Fig. 2 is a western blot showing EphA2 expression in various human mammary epithelial cell lines;
Fig. 3A-B shows EphA2 localization in the cell membranes of various mammary epithelial cell lines, as seen by immunofluorescence microscopy;

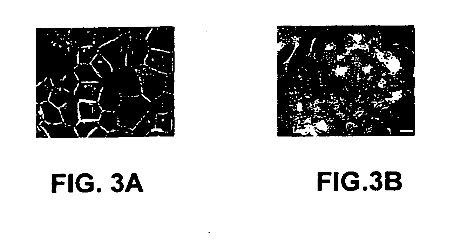

Fig. 3A shows EphA2 localization in sites of cell adhesion in normal MCF-10A cells; and
Fig. 3B shows EphA2 redistribution in malignant cells.

### Detailed Description of the Invention

Antibodies specific for EphA2 have been isolated through an improved method. The method employed is designed for increased sensitivity and diversity of responding hybridomas. According to this method, tyrosine phosphorylated proteins from Ras-transformed human epithelial cells are isolated by affinity chromatography using existing phosphotyrosine-specific antibodies. The tyrosine phosphorylated proteins are then used as an immunogen for producing monoclonal antibodies. Low-dose amounts of tyrosine phosphorylated proteins are injected proximal to lymph nodes, every other day, over a ten day period (the RIMMS strategy). B cells from engorged lymph nodes are then isolated and fused with a Bcl-2-overexpressing myeloma, to minimize apoptosis after fusion. This method results in increased diversity, specificity, and cost-effectiveness of hybridoma production. The hybridomas are first screened to identify those hybridomas producing antibodies capable of distinguishing malignant from normal cancer cells. To date, at least 450 such hybridomas have been identified.

Hybridomas which are specific to EphA2 have been selected. Use of the RIMMS strategy has resulted in the production of various monoclonal antibodies that specifically bind EphA2. Of the first four hybridomas characterized, two recognize independent epitopes on EphA2. The first, D7, recognizes an intracellular epitope. The second, B2D6, binds to an extracellular epitope. D7 has proven to be highly specific for an intracellular epitope of EphA2 and this specificity provides much of the current basis for diagnosis of metastatic tumors.

It is known in the art to use antibodies to detect the presence or overexpression of a specific protein. Because EphA2 is overexpressed in metastatic cells, EphA2-specific antibodies of this invention may be used to detect this overexpression and, thus, to detect metastatic disease. Such techniques include but are not limited to western blotting, dot blotting, precipitation, agglutination, ELISA assays, immunohistochemistry, in situ hybridization, flow cytometry on a variety of tissues or bodily fluids, and a variety of sandwich assays. These techniques are well known in the art. See, for example, U.S. Patent No. 5,876,949, hereby incorporated by reference. When antibodies specific for an intracellular epitope of EphA2 are used, the cells must be lysed and incubated with the antibody. The above techniques may be performed on whole-cell lysates, or EphA2 may be separated out for testing, such as by immunoprecipitation. The D7 antibodies of this invention are highly specific for an intracellular epitope of EphA2 and have proven to be sensitive to differential expression of EphA2 in metastatic cells. Other techniques, such as immunohistological staining, require whole cells, and may further require cell layers of a particular cell density. Such tests require an antibody specific for an extracellular epitope of EphA2.

The antibodies of this invention may be used to detect metastatic disease in a wide variety of tissue samples. For instance, research using EphA2-specific antibodies has revealed that altered EphA2 expression occurs in breast, kidney, prostate, lung, and colon cells, and it is believed that altered EphA2 expression occurs in other types of cell metastasis, particularly epithelial malignancies. EphA2-specific antibodies may be used to detect metastasis in biopsied tumor tissue. Also, samples of a variety of body fluid samples, such as blood, plasma, spinal fluid, saliva, and urine, can be tested with the antibodies of the present invention. Altered EphA2 expression in these samples indicates the presence of metastatic disease.

Additionally, other antibodies may be used in combination with the antibodies of the present invention to provide further information concerning metastatic disease state. For example, the EphA2 of metastatic cells exhibits altered tyrosine phosphorylation. In normal breast epithelial cells, EphA2 is expressed and is tyrosine phosphorylated. However, in metastatic breast epithelial cells, EphA2 is overexpressed, and the EphA2 is not tyrosine phosphorylated. Because a test quantifying EphA2 expression sometimes may lead to an ambiguous result, it may be desirable to determine tyrosine phosphorylation, as well as the magnitude of EphA2 expression. Thus, a method of diagnosis using the antibodies of this invention in combination with phosphotyrosine-specific antibodies provides data for determining the state of metastatic disease.

Moreover, the EphA2-specific antibodies of this invention can be exploited to detect changes in EphA2 localization which are associated with metastasis. In normal breast and prostate epithelial cells, EphA2 is enriched in within sites of cell adhesion. Conversely, in metastatic prostate cells EphA2 is diffusely distributed, and in metastatic breast cancer cells EphA2 is redistributed into the membrane ruffles. Techniques such as immunohistological staining or immunofluorescent microscopy are well known in the art and may be used to visualize EphA2 distribution. See, for example, U.S. Patent No. 5,514,554, hereby incorporated by reference. EphA2 expression can be detected by using antibodies capable of detecting whole EphA2 or fragments of the EphA2 protein. Other methods of detecting altered EphA2 expression include detecting DNA or RNA sequences coding for the EphA2 protein.

In order to detect overexpression or altered distribution of EphA2, the EphA2-specific antibodies may be labeled covalently or non-covalently with any of a number of known detectable labels, such fluorescent, radioactive, or enzymatic substances, as is known in the art. Alternatively, a secondary antibody specific for the antibodies of this invention is labeled with a known detectable label and used to detect the EphA2-specific antibodies in the above techniques.

Preferred labels include chromogenic dyes. Among the most commonly used are 3-amino-9-ethylcarbazole (AEC) and 3,3'-diaminobenzidine tetrahydrocholoride (DAB). These can be detected using light microscopy. Also preferred are fluorescent labels. Among the most commonly used fluorescent labeling compounds are fluorescein isothiocyanate, rhodamine, phycoerythrin, phycocyanin, allophycocyanin, o-phthaldehyde and fluorescamine. Chemiluminescent and bioluminescent compounds such as luminol, isoluminol, theromatic acridinium ester, imidazole, acridinium salt, oxalate ester, luciferin, luciferase, and aequorin also may be used. When the fluorescent-labeled antibody is exposed to light of the proper wavelength, its presence can be detected due to its fluorescence.

Also preferred are radioactive labels. Radioactive isotopes which are particularly useful for labeling the antibodies of the present invention include ³H, ¹²⁵I, ¹³¹I, ³⁵S, ³²P, and ¹⁴C. The radioactive isotope can be detected by such means as the use of a gamma counter, a scintillation counter, or by autoradiography.

Another method in which the antibodies can be detectably labeled is by linking the antibodies to an enzyme and subsequently using the antibodies in an enzyme immunoassay (EIA) or enzyme-linked immunosorbent assay (ELISA). The enzyme, when subsequently exposed to its substrate, reacts with the substrate and generates a chemical moiety which can be detected, for example, by spectrophotometric, fluorometric, or visual means. Enzymes which can be used to detectably label antibodies include, but are not limited to malate dehydrogenase, staphylococcal nuclease, delta-5-steroid isomerase, yeast alcohol dehydrogenase, alpha-glycerophosphate dehydrogenase, triose phosphate isomerase, horseradish peroxidase, alkaline phosphatase, asparaginase, glucose oxidase, beta-galactosidase, ribonuclease, urease, catalase, glucose-6-phosphate dehydrogenase, glucoamylase, and acetylcholinesterase. Other methods of labeling and detecting antibodies are known in the art and are within the scope of this invention.

### Example 1

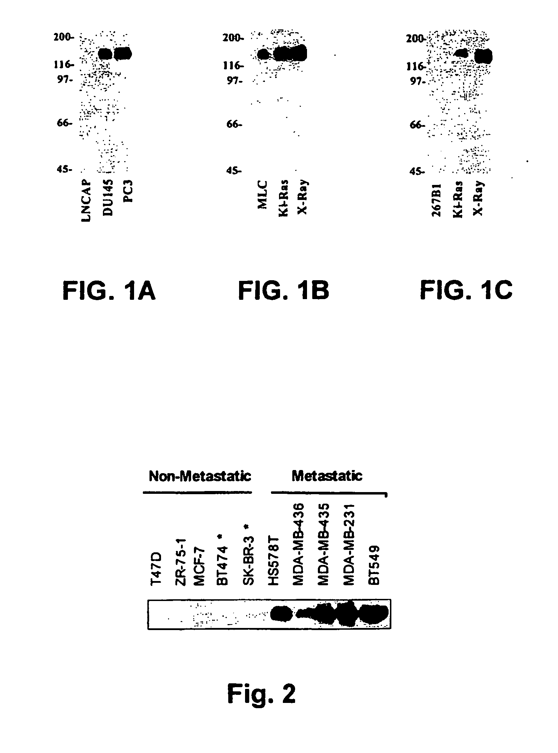

The antibodies produced by the D7 hybridoma are used to detect differential expression of EphA2 between normal prostate epithelial cells and metastatic cells. Fig. 1 shows EphA2 expression in various human prostate cell lines. Referring first to Fig. 1A, three metastatic cell lines, LNCAP, DU145, and PC3, are tested for levels of EphA2 expression. It is known that, of these three cell lines, LNCAP is the least invasive, DU145 is somewhat more invasive, and PC3 is the most invasive. EphA2 expression is determined by western blotting with D7 antibodies. As can be seen in Fig. 1A, EphA2 expression positively correlates with invasiveness.

In Fig. 1B, D7 antibodies are used to test EphA2 expression in normal MLC cells as compared to expression in transformed cells. Normal MLC cells, MLC cells which have been transformed by K-Ras, and MLC cells with have been transformed by X-irradiation are studied. As can be seen in Fig. 1B, EphA2 is overexpressed in both of the transformed cell lines. Fig. 1C shows results similar to Fig. 1B, except the normal cell line is 267B1. As with Fig. 1B, Fig. 1C shows that EphA2 is overexpressed in the transformed cells. In sum, Fig. 1 demonstrates that EphA2-specific antibodies detect changes in metastatic cells, and that tests using these antibodies indicate the level of metastatic invasiveness.

### Example 2

EphA2 antibodies are used to detect altered EphA2 expression in metastatic mammary cells. EphA2 is expressed in normal mammary epithelial cells. Fig. 2 illustrates altered EphA2 expression in mammary tumor cell lines. As can be seen in Fig. 2, western blots from whole cell lysates using D7 antibodies reveal that EphA2 expression is completely absent in cells derived from non-metastatic breast tumors (ZR75-1, BT474, SKBR3, MDA-MB-435). By contrast, EphA2 is overexpressed in metastatic breast cancer cell lines (MDA-MB-435, MDA-MB-231). Thus, EphA2 antibodies detect altered EphA2 expression in breast cancer cells, which can be used to diagnose metastasis. Moreover, in non-metastatic breast epithelial cells, loss of EphA2 occurs early in the disease, and testing with EphA2-specific antibodies provide information relevant to invasiveness even when other known markers remain normal. Thus, D7 antibodies are useful as a diagnostic, even in early stages of disease.

### Example 3

EphA2 antibodies in combination with other antibodies are used to detect further alterations in EphA2 expression. As discussed above in Example 2, western blots using D7 can distinguish between non-metastatic and metastatic tumors, with non-metastatic tumors failing to express EphA2, and metastatic cells overexpressing EphA2. However, different results are found when tyrosine phosphorylation is studied. Using a phosphotyrosine-specific antibody, it has been found that EphA2 is phosphorylated in normal cells, but it is not phosphorylated in metastatic cells. Thus, while EphA2 specific antibodies can qualitatively detect a difference between metastatic and non-metastatic mammary tumor cells, diagnostics incorporating both an EphA2-specific antibody and a phosphotyrosine-specific antibody provides a sensitive test for distinguishing between normal, non-metastatic, and metastatic mammary cells.

### Example 4

Immunofluorescently labeled EphA2-specific antibodies detect redistribution of EphA2 expression in transformed cells. The EphA2-specific antibodies used in this example are produced by a cell line known as B2D6, and these antibodies are specific for an extracellular epitope of EphA2. As seen in Fig. 3A, immunofluorescence with B2D6 demonstrates that EphA2 is found within sites of cell-cell contact in normal cells. However, in transformed cells, shown in Fig. 3B, EphA2 is redistributed. Furthermore, in metastatic cells EphA2 is found in the membrane ruffles. Similarly, in normal prostate epithelial cells, EphA2 is found within sites of cell-cell adhesion, but in metastatic prostate epithelial cells, EphA2 is overexpressed and the expression is diffusely distributed. Therefore, immunofluorescence using EphA2-specific antibodies provides an additional means for diagnosing the transformation and metastatic state of tumor cells.

As shown in Examples 1-4, overexpression, redistribution, and phosphorylation of EphA2 in metastatic cells provide various bases for diagnosis of metastatic tumors using EphA2-specific antibodies. Immunohistochemistry or Western blotting may be used to monitor the change of EphA2 expression in biopsied samples of patient breast tissue, prostate tissue, or tissue from other tumors. Additionally, D7 and other EphA2-specific antibodies can be used to monitor plasma, urine, and other body fluids to detect altered expression of EphA2, which would signal metastasis. Detection of altered tyrosine phosphorylation of EphA2 in combination with information concerning an alteration of EphA2 expression further aids in diagnosis of metastatic disease.

Although the invention has been described in detail with reference to preferred embodiments, variations and modifications exist within the scope of the invention as described and defined in the following claims.

## Claims

1. An in vitro method of detecting the presence of metastatic cancer cells in a selected cell population comprising:
assaying at least a portion of the selected cell population for EphA2 overexpression, wherein overexpression is indicative of the presence of a metastatic cancer cell in the selected cell population.

2. The method of claim 1 further comprising determining the state of metastatic disease in the cell population.

3. The in vitro method of claim 1 comprising
(a) incubating the cells with
(i) a reagent capable of specific binding to EphA2; or
(ii) a reagent capable of specific binding to an RNA encoding at least a portion of an EphA2 protein
to allow binding of the reagent to EphA2 or the RNA;
(b) detecting reagent binding to EphA2 or the RNA; and
(c) detecting overexpression of EphA2, wherein overexpression of EphA2 is indicative of the presence of metastatic or potentially metastatic cells in the cell population.

4. The method of claim 3 further comprising lysing at least a portion of the cell population prior to incubation with the reagent.

5. The method of any of the preceding claims wherein the reagent is an antibody.

6. The method of claim 5 wherein the antibody is capable of specific binding to an intracellular epitope of EphA2.

7. The method of claim 5 or 6 wherein the antibody is produced by hybridoma cell line D7.

8. The method of claim 5 wherein the antibody is capable of specific binding to an extracellular epitope of EphA2.

9. The method of claim 8 wherein the antibody is produced by hybridoma cell line B2D6.

10. The method of any one of claims 5 to 9 wherein the reagent is labeled with at least one detectable label, and the detecting step includes detecting the label.

11. The method of claim 10 wherein the detectable label is selected from the group consisting of a fluorescent label, a chemiluminescent label, a bioluminescent label, an enzymatic label, a chromogenic label and a radiolabel.

12. The method of claims 3 to 11 wherein the detecting step includes a diagnostic method selected from the group consisting of ELISA assays and flow cytometry.

13. The method of any one of claims 5 to 11 wherein the incubating and detecting steps comprise western blotting methodology.

14. The method of claim 13 further comprising the steps of providing a second antibody having phosphotyrosine specificity, and western blotting with the second antibody.

15. The method of claim 3 wherein the reagent binding yields a bound complex comprising a whole cell.

16. The method of claim 15 wherein detecting reagent binding comprises subjecting the bound complex to immunohistochemical staining.

17. The method of claim 3 further comprising the step of fixing the cells on a slide, and the detecting step comprises immunofluorescence staining.

18. The method of any of the preceding claims wherein the cell population comprises cells selected from the group consisting of breast cells, kidney cells, prostate cells, lung cells and colon cells.

19. The method of any of the preceding claims wherein the cell population comprises epithelial cells.

20. The method of any of the preceding claims wherein the cell population comprises cells selected from the group consisting of breast cancer cells, kidney cancer cells, prostate cancer cells, lung cancer cells and colon cancer cells.

21. The method of any of the preceding claims wherein the cell population comprises epithelial cancer cells.

22. The method of any of the preceding claims wherein the metastatic cancer cells comprise cells selected from the group consisting of breast cancer cells, kidney cancer cells, prostate cancer cells, lung cancer cells and colon cancer cells.

23. The method of any of the preceding claims wherein the metastatic cancer cells comprise epithelial cancer cells

24. The method of any of the preceding claims wherein the cell population comprises cells from a tissue biopsy.

25. The method of any of the preceding claims wherein the cell population comprises cells from a breast or prostate tissue biopsy.

26. The method of any one of claims 1 or 23 wherein the cell population comprises cells from a body fluid.

27. The method of claim 26 wherein the cells are harvested from a body fluid selected from the group consisting of blood, plasma, spinal fluid, saliva, and urine.

28. The method of claim 1 wherein assaying the cell population comprises incubating at least a portion of the cancer cell population with a reagent capable of binding to EphA2 to allow binding of the reagent to EphA2; and detecting reagent binding.

29. The method of claim 28 wherein the reagent is an antibody.

30. The method of claim 29 wherein the antibody is produced by hybridoma D7 or B2D6.

31. Use of an antibody capable of specific binding to an epitope of EphA2 and means for detecting said antibody-epitope binding for detecting the presence of metastatic cells in a cell population.

32. The use of claim 31 wherein the means for detecting antibody-epitope binding is a label bound to the antibody.

33. The use of claim 31 or 32 further comprising an antibody having phosphotyrosine specificity.

34. A method for distinguishing between metastatic and non-metastatic cancers, comprising determining EphA2 expression with anti-EphA2 antibodies, wherein EphA2 overexpression indicates that the cancer is metastatic, and absence of EphA2 expression indicates that the cancer is non-metastatic.

## Patentansprüche

1. *In vitro*-Verfahren zum Nachweis der Anwesenheit von metastatischen Krebszellen in einer ausgewählten Zellpopulation, umfassend:
das Untersuchen mindestens eines Teils der ausgewählten Zellpopulation auf EphA2-Überexpression, wobei Überexpression die Anwesenheit einer metastatischen Krebszelle in der ausgewählten Zellpopulation anzeigt.

2. Verfahren nach Anspruch 1, ferner umfassend die Bestimmung des Status' der metastatischen Krankheit in der Zellpopulation.

3. *In vitro*-Verfahren nach Anspruch 1, umfassend
(a) Inkubieren der Zellen mit
(i) einem Reagenz, welches zur spezifischen Bindung an EphA2 fähig ist; oder
(ii) einem Reagenz, welches zur spezifischen Bindung an eine RNA fähig ist, die mindestens einen Teil eines EphA2-Proteins codiert,
um Bindung des Reagenz' mit EphA2 oder der RNA zu ermöglichen;
(b) Nachweis der Reagenzbindung an EphA2 oder an die RNA; und
(c) Nachweis von EphA2-Überexpression, wobei Überexpression von EphA2 die Anwesenheit von metastatischen oder potentiell metastatischen Zellen in der Zellpopulation anzeigt.

4. Verfahren nach Anspruch 3, ferner umfassend das Lysieren mindestens eines Teils der Zellpopulation vor der Inkubation mit dem Reagenz.

5. Verfahren nach einem der vorhergehenden Ansprüche, wobei das Reagenz ein Antikörper ist.

6. Verfahren nach Anspruch 5, wobei der Antikörper zur spezifischen Bindung an ein intrazelluläres Epitop von EphA2 fähig ist.

7. Verfahren nach Anspruch 5 oder 6, wobei der Antikörper durch die Hybridomzelllinie D7 hergestellt wird.

8. Verfahren nach Anspruch 5, wobei der Antikörper zur spezifischen Bindung an ein extrazelluläres Epitop von EphA2 fähig ist.

9. Verfahren nach Anspruch 8, wobei der Antikörper durch die Hybridomzelllinie B2D6 hergestellt wird.

10. Verfahren nach einem der Ansprüche 5 bis 9, wobei das Reagenz mit mindestens einem nachweisbaren Marker markiert ist, und der Nachweisschritt das Nachweisen des Markers beinhaltet.

11. Verfahren nach Anspruch 10, wobei der nachweisbare Marker ausgewählt ist aus der Gruppe bestehend aus einem Fluoreszenzmarker, einem Chemilumineszenzmarker, einem Biolumineszenzmarker, einem enzymatischen Marker, einem chromogenen Marker und einem Radiomarker.

12. Verfahren nach Ansprüchen 3 bis 11, wobei der Nachweisschritt ein diagnostisches Verfahren ausgewählt aus der Gruppe bestehend aus ELISA-Untersuchungen und Durchflusszytometrie beinhaltet.

13. Verfahren nach einem der Ansprüche 5 bis 11, wobei die Inkubations- und Nachweisschritte Western-Blotting-Verfahren umfassen.

14. Verfahren nach Anspruch 13, ferner umfassend die Schritte der Bereitstellung eines zweiten Antikörpers mit Phosphotyrosin-Spezifität und eines Western-Blots mit dem zweiten Antikörper.

15. Verfahren nach Anspruch 3, wobei die Reagenzbindung einen gebundenen Komplex umfassend eine ganze Zelle ergibt.

16. Verfahren nach Anspruch 15, wobei das Nachweisen von Reagenzbindung das Durchführen einer immunhistochemischen Färbung mit dem gebundenen Komplex umfasst.

17. Verfahren nach Anspruch 3, ferner umfassend den Schritt der Fixierung der Zellen auf einem Objektträger, und der Nachweisschritt umfasst Immunofluoreszenzfärbung.

18. Verfahren nach einem der vorhergehenden Ansprüche, wobei die Zellpopulation Zellen ausgewählt aus der Gruppe bestehend aus Brustzellen, Nierenzellen, Prostatazellen, Lungenzellen und Darmzellen umfasst.

19. Verfahren nach einem der vorhergehenden Ansprüche, wobei die Zellpopulation Epithelzellen umfasst.

20. Verfahren nach einem der vorhergehenden Ansprüche, wobei die Zellpopulation Zellen ausgewählt aus der Gruppe bestehend aus Brustkrebszellen, Nierenkrebszellen, Prostatakrebszellen, Lungenkrebszellen und Darmkrebszellen umfasst.

21. Verfahren nach einem der vorhergehenden Ansprüche, wobei die Zellpopulation Epithelkrebszellen umfasst.

22. Verfahren nach einem der vorhergehenden Ansprüche, wobei die metastatischen Krebszellen Zellen ausgewählt aus der Gruppe bestehend aus Brustkrebszellen, Nierenkrebszellen, Prostatakrebszellen, Lungenkrebszellen und Darmkrebszellen umfassen.

23. Verfahren nach einem der vorhergehenden Ansprüche, wobei die metastatischen Krebszellen Epithetkrebszellen umfassen.

24. Verfahren nach einem der vorhergehenden Ansprüche, wobei die Zellpopulation Zellen aus einer Gewebsbiopsie umfasst.

25. Verfahren nach einem der vorhergehenden Ansprüche, wobei die Zellpopulation Zellen aus einer Brust- oder Prostata-Gewebsbiopsie umfasst.

26. Verfahren nach einem der Ansprüche 1 oder 23, wobei die Zellpopulation Zellen aus einer Körperflüssigkeit umfasst.

27. Verfahren nach Anspruch 26, wobei die Zellen von einer Körperflüssigkeit ausgewählt aus der Gruppe bestehend aus Blut, Plasma, Rückenmarksflüssigkeit, Speichel und Urin geerntet werden.

28. Verfahren nach Anspruch 1, wobei das Untersuchen der Zellpopulation das Inkubieren mindestens eines Teils der Zellpopulation der Krebszellpopulation mit einem Reagenz umfasst, welches zur Bindung mit EphA2 fähig ist, um die Bindung des Reagenz' an EphA2 zu ermöglichen; und Nachweisen der Reagenzbindung.

29. Verfahren nach Anspruch 28, wobei das Reagenz ein Antikörper ist.

30. Verfahren nach Anspruch 29, wobei der Antikörper durch Hybridom D7 oder B2D6 hergestellt wird.

31. Verwendung eines Antikörpers, welcher zur spezifischen Bindung an ein Epitop von EphA2 fähig ist und von Mitteln zum Nachweis der Antikörper-Epitop-Bindung zum Nachweis der Anwesenheit von metastatischen Zellen in einer Zellpopulation.

32. Verwendung nach Anspruch 31, wobei das Mittel zum Nachweis der Antikörper-Epitop-Bindung ein an den Antikörper gebundener Marker ist.

33. Verwendung nach Anspruch 31 oder 32, ferner umfassend einen Antikörper mit Phosphotyrosin-Spezifität.

34. Verfahren zur Unterscheidung zwischen metastatischem und nichtmetastatischem Krebs, umfassend das Bestimmen von EphA2-Expression mit Anti-EphA2-Antikörpern, wobei EphA2-Überexpression anzeigt, dass der Krebs metastatisch ist, und Abwesenheit von EphA2-Expression anzeigt, dass der Krebs nicht-metastatisch ist.

## Revendications

1. Procédé *in vitro* permettant de détecter la présence de cellules cancéreuses métastasiques dans une population cellulaire choisie, comprenant :
l'analyse d'au moins une partie de la population cellulaire choisie pour une surexpression de l'EphA2, où une surexpression indique la présence d'une cellule cancéreuse métastasique dans la population cellulaire choisie.

2. Procédé selon la revendication 1, comprenant en outre la détermination de l'état de la maladie métastasique dans la population cellulaire.

3. Procédé *in vitro* selon la revendication 1 comprenant
(a) l'incubation des cellules avec
(i) un réactif capable de liaison spécifique à l'EphA2 ; ou
(ii) un réactif capable de liaison spécifique à un ARN codant pour au moins une partie d'une protéine EphA2 pour permettre la liaison du réactif à l'EphA2 ou à l'ARN ;
(b) la détection de la liaison du réactif à l'EphA2 ou à l'ARN ; et
(c) la détection de la surexpression de l'EphA2, où une surexpression de l'EphA2 indique la présence de cellules métastasiques ou potentiellement métastasiques dans la population cellulaire.

4. Procédé selon la revendication 3, comprenant en outre la lyse d'au moins une partie de la population cellulaire avant l'incubation avec le réactif.

5. Procédé selon l'une quelconque des revendications précédentes, dans lequel le réactif est un anticorps.

6. Procédé selon la revendication 5, dans lequel l'anticorps est capable de liaison spécifique à un épitope intracellulaire de l'EphA2.

7. Procédé selon la revendication 5 ou 6, dans lequel l'anticorps est produit par la lignée cellulaire d'hybridome D7.

8. Procédé selon la revendication 5, dans lequel l'anticorps est capable de liaison spécifique à un épitope extracellulaire de l'EphA2.

9. Procédé selon la revendication 8, dans lequel l'anticorps est produit par la lignée cellulaire d'hybridome B2D6.

10. Procédé selon l'une quelconque des revendications 5 à 9, dans lequel le réactif est marqué avec au moins un marqueur détectable et l'étape de détection comprend la détection du marqueur.

11. Procédé selon la revendication 10, dans lequel le marqueur détectable est choisi dans le groupe constitué par un marqueur fluorescent, un marqueur chimioluminescent, un marqueur bioluminescent, un marqueur enzymatique, un marqueur chromogène et un radio-marqueur.

12. Procédé selon les revendications 3 à 11, dans lequel l'étape de détection comprend un procédé de diagnostic choisi dans le groupe constitué par des tests ELISA et la cytométrie en flux.

13. Procédé selon l'une quelconque des revendications 5 à 11, dans lequel les étapes d'incubation et de détection comprennent la méthodologie Western blot.

14. Procédé selon la revendication 13, comprenant en outre les étapes de fourniture d'un second anticorps présentant une spécificité pour la phosphotyrosine et d'analyse Western blot avec le second anticorps.

15. Procédé selon la revendication 3, dans lequel la liaison du réactif produit un complexe lié comprenant une cellule entière.

16. Procédé selon la revendication 15, dans lequel la détection de la liaison du réactif comprend la soumission du complexe lié à une coloration immunohistochimique.

17. Procédé selon la revendication 3, comprenant en outre l'étape de fixation des cellules sur une lame et l'étape de détection comprend une coloration d'immunofluorescence.

18. Procédé selon l'une quelconque des revendications précédentes, dans lequel la population cellulaire comprend des cellules choisies dans le groupe constitué par des cellules du sein, des cellules du rein, des cellules de la prostate, des cellules du poumon et des cellules du côlon.

19. Procédé selon l'une quelconque des revendications précédentes, dans lequel la population cellulaire comprend des cellules épithéliales.

20. Procédé selon l'une quelconque des revendications précédentes, dans lequel la population cellulaire comprend des cellules choisies dans le groupe constitué par des cellules cancéreuses du sein, des cellules cancéreuses du rein, des cellules cancéreuses de la prostate, des cellules cancéreuses du poumon et des cellules cancéreuses du côlon.

21. Procédé selon l'une quelconque des revendications précédentes, dans lequel la population cellulaire comprend des cellules épithéliales cancéreuses.

22. Procédé selon l'une quelconque des revendications précédentes, dans lequel les cellules cancéreuses métastasiques comprennent des cellules choisies dans le groupe constitué par des cellules cancéreuses du sein, des cellules cancéreuses du rein, des cellules cancéreuses de la prostate, des cellules cancéreuses du poumon et des cellules cancéreuses du côlon.

23. Procédé selon l'une quelconque des revendications précédentes, dans lequel les cellules cancéreuses métastasiques comprennent des cellules épithéliales cancéreuses.

24. Procédé selon l'une quelconque des revendications précédentes, dans lequel la population cellulaire comprend des cellules provenant d'une biopsie tissulaire.

25. Procédé selon l'une quelconque des revendications précédentes, dans lequel la population cellulaire comprend des cellules provenant d'une biopsie de tissu du sein ou de la prostate.

26. Procédé selon l'une quelconque des revendications 1 à 23, dans lequel la population cellulaire comprend des cellules provenant d'un liquide corporel.

27. Procédé selon la revendication 26, dans lequel les cellules sont récoltées à partir d'un liquide corporel choisi dans le groupe constitué par du sang, du plasma, du liquide rachidien, de la salive et de l'urine.

28. Procédé selon la revendication 1, dans lequel l'analyse de la population cellulaire comprend l'incubation d'au moins une partie de la population des cellules cancéreuses avec un réactif capable de se lier à l'EphA2 pour permettre la liaison du réactif à l'EphA2 ; et la détection de la liaison du réactif.

29. Procédé selon la revendication 28, dans lequel le réactif est un anticorps.

30. Procédé selon la revendication 29, dans lequel l'anticorps est produit par l'hybridome D7 ou B2D6.

31. Utilisation d'un anticorps capable de liaison spécifique à un épitope de l'EphA2 et d'un moyen pour détecter ladite liaison anticorps-épitope pour la détection de la présence de cellules métastasiques dans une population cellulaire.

32. Utilisation selon la revendication 31, dans lequel le moyen pour détecter la liaison anticorps-épitope est un marqueur lié à l'anticorps.

33. Utilisation selon la revendication 31 ou 32, comprenant en outre un anticorps présentant une spécificité pour la phosphotyrosine.

34. Procédé permettant de faire la distinction entre des cancers métastasiques et non métastasiques, comprenant la détermination de l'expression de l'EphA2 avec des anticorps anti-EphA2, où une surexpression de l'EphA2 indique que le cancer est métastasique et l'absence d'expression de l'EphA2 indique que le cancer est non métastasique.
